Europäisches Patentamt

European Patent Office

Office européen des brevets

(19)

(11) Numéro de publication: **0 172 079**

**A1**

## (12) DEMANDE DE BREVET EUROPEEN

(21) Numéro de dépôt: 85401462.8

(22) Date de dépôt: 17.07.85

(51) Int. Cl.⁴: **B 05 B 7/30**
**A 61 M 11/04**

(30) Priorité: 25.07.84 FR 8411789

(43) Date de publication de la demande:
19.02.86 Bulletin 86/8

(84) Etats contractants désignés:
BE DE FR GB NL

(71) Demandeur: SEB S.A.

F-21260 Selongey(FR)

(72) Inventeur: Hennuy, Jean
En Machon Chervinges
F-69400 Villefranche/Saone(FR)

(72) Inventeur: Seguret, René
1, rue du Vieux Cep Limas
F-69400 Villefranche/Saone(FR)

(74) Mandataire: Bouju, André
Cabinet Bouju 38 avenue de la Grande Armée
F-75017 Paris(FR)

(54) Distributeur pour vaporisateur-diffuseur à vapeur.

(57) Le distributeur pour vaporisateur-diffuseur à vapeur selon l'invention comporte un manchon (4) comprenant un orifice d'alimentation (5) de vapeur, un corps creux (6) disposé dans le manchon (4) et comportant un orifice de liaison (9) entre l'intérieur du corps (6) et l'intérieur du manchon (4), et au moins un premier et un second orifices de sortie (10, 12) distincts dont l'un (10) est équipé d'une buse de vaporisation (11), au moins un clapet de séparation (13) entre les orifices de sortie (10, 12), et un organe de commande (16) de la position du clapet (13).

FIG_3

EP 0 172 079 A1

"Distributeur pour vaporisateur-diffuseur à vapeur"

La présente invention concerne un distributeur pour vaporisateur-diffuseur à vapeur.

On connaît des vaporisateurs, par exemple des vaporisateurs de lotion, dans lesquels le produit à vaporiser est aspiré par un jet de vapeur dirigé sensiblement perpendiculairement à un conduit d'amenée du produit à vaporiser. Dans ce cas, on dispose généralement d'une possibilité de diffuser séparément de la vapeur pour un usage différent de la vaporisation. En particulier, dans le cas d'un appareil de traitement de la peau et plus spécifiquement de la peau du visage, il est souhaitable de disposer d'une source de vapeur pure généralement destinée à provoquer une dilatation des pores de la peau, la vaporisation d'une lotion froide étant ensuite utilisée pour rafraîchir le visage.

Toutefois, dans les dispositifs connus, la fonction de diffusion de la vapeur pure et la fonction de vaporisation sont nettement séparées et ces dispositifs comprennent donc des moyens de mise en oeuvre séparés de sorte que leur utilisation requiert une série de manipulations pour passer d'une fonction à une autre.

Un premier but de la présente invention est donc de proposer un distributeur pour vaporisateur-diffuseur à vapeur ayant une structure permettant de passer aisément de la fonction de diffusion de la vapeur pure à la fonction de vaporisation, et vice-versa, avec un minimum de manipulations.

Par ailleurs, les dispositifs de vaporisation et de diffusion de la vapeur comportent généralement des buses ayant une orientation unique de sorte que l'ensemble de l'appareil doit être incliné pour suivre les contours du visage ce qui pose parfois des problèmes d'aspiration de la lotion surtout lorsqu'il reste une faible quantité de celle-ci dans l'appareil.

Un autre but de la présente invention est de proposer un distributeur pour vaporisateur-diffuseur à vapeur ayant une structure simple permettant de changer l'orientation du jet de vapeur ou du jet de produit vaporisé sans nécessairement modifier la position de l'appareil.

En vue de la réalisation du premier but de l'invention, on prévoit un distributeur pour vaporisateur-diffuseur à vapeur caractérisé en ce qu'il comporte un manchon comprenant un orifice d'alimentation de vapeur, un corps creux disposé dans le manchon et comportant un orifice de liaison entre l'intérieur du corps creux et l'intérieur du manchon et au moins un premier et un second orifices de sortie distincts dont l'un est équipé d'une buse de vaporisation, au moins un clapet de séparation entre les orifices de sortie, et un organe de commande de la position du clapet.

Ainsi, par la simple manoeuvre du clapet au moyen de l'organe de commande, on passe aisément de la fonction de vaporisation à la fonction de diffusion de vapeur pure et vice versa.

Selon une version avantageuse de l'invention visant à la réalisation du second but, le corps creux est monté pivotant dans le manchon et sa position est contrôlée par un organe de commande commun à l'organe de commande de la position du clapet.

Ainsi, par la manoeuvre du même organe de commande il est possible de modifier l'orientation du jet de produit vaporisé ou de vapeur tout en changeant simultanément de fonction.

Selon une réalisation préférée de l'invention, l'organe de commande est une tige coulissante comprenant des moyens de solidarisation pour une rotation avec le corps creux, la tige ayant une extrémité reliée au clapet et l'autre extrémité portant une poignée de commande. Ainsi, par une traction sur la poignée de commande, on provoque un basculement de la fonction de vaporisation à la fonction de distribution de vapeur pure ou vice versa et par une rotation de la poignée de commande on modifie l'orientation du jet de produit vaporisé ou de vapeur pure.

Selon un autre aspect de la réalisation préférée de l'invention, la poignée de commande est montée de façon pivotante autour d'un axe transversal à la tige de commande et comporte des flasques s'étendant transversalement à cet axe, ces flasques ayant un bord curviligne excentré par rapport à l'axe de pivotement de la poignée de commande, ce bord prenant appui sur un organe de butée solidaire du corps creux. Ainsi, par un basculement de la poignée de commande autour de son axe de pivotement, on met en oeuvre la fonction désirée qui est ainsi maintenue tant que l'orientation de la poignée de commande n'est pas modifiée.

Selon un autre aspect de la réalisation préférée de l'invention, les moyens de solidarisation en rotation entre la tige et le corps creux comprennent des ergots portés par la tige, s'étendant transversalement à celle-ci et coopérant avec des rainures portées par l'organe de butée. Ainsi on obtient un coulissement de la tige avec de très faibles frottements et donc une manipulation aisée de la commande.

Selon encore un autre aspect de la réalisation préférée de l'invention, le clapet est disposé entre l'orifice de liaison de l'intérieur du corps creux avec le manchon et le second orifice de sortie, et le second orifice de sortie a une dimension supérieure à un canal de vapeur de la buse, la buse étant portée par le premier orifice. Ainsi, lorsque le clapet est ouvert, la perte de charge du second orifice de sortie est inférieure à celle du premier orifice de sortie et l'on obtient un arrêt de la vaporisation sans qu'il soit nécessaire de prévoir un dispositif de fermeture du premier orifice de sortie.

D'autres caractéristiques et avantages de l'invention résulteront encore de la description ci-après d'un exemple non limitatif en référence aux dessins annexés dans lesquels:

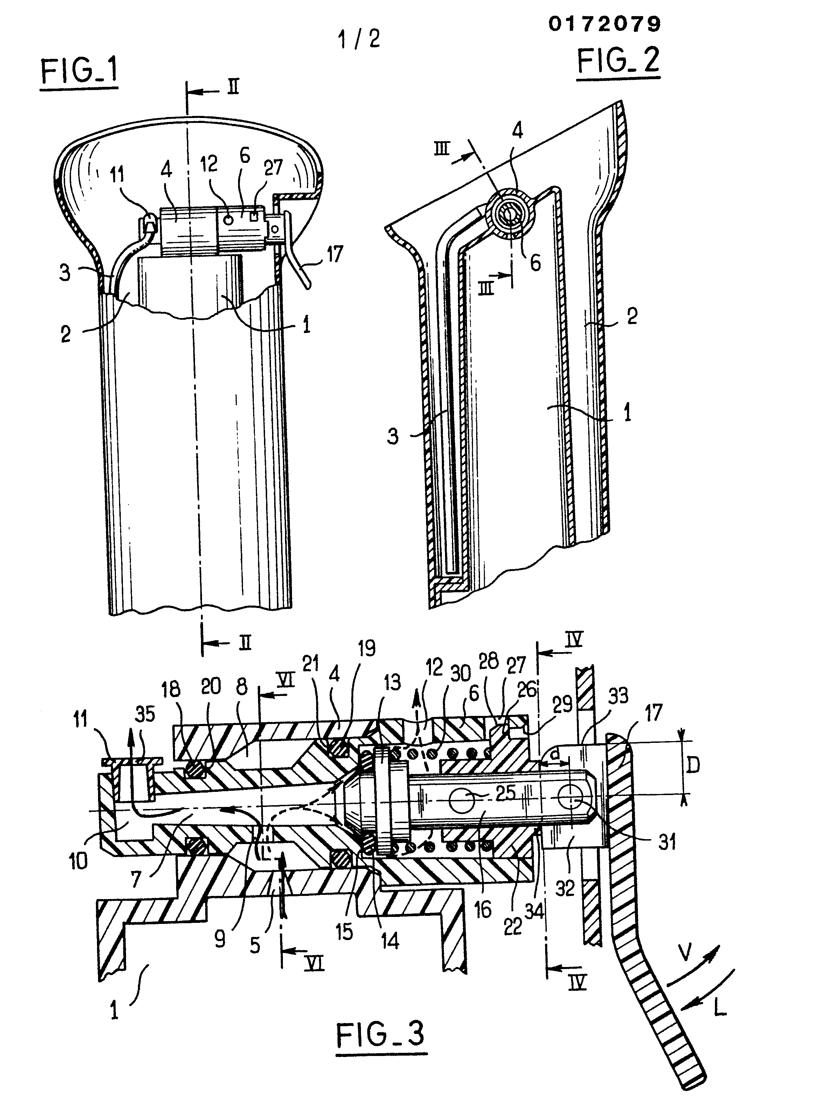

. la figure 1 est une vue partielle, partiellement arrachée, d'un vaporisateur-diffuseur à vapeur comprenant le distributeur selon l'invention;

. la figure 2 est une vue en coupe selon le plan II-II de la figure 1;

. la figure 3 est une vue en coupe agrandie du distributeur selon le plan III-III de la figure 2;

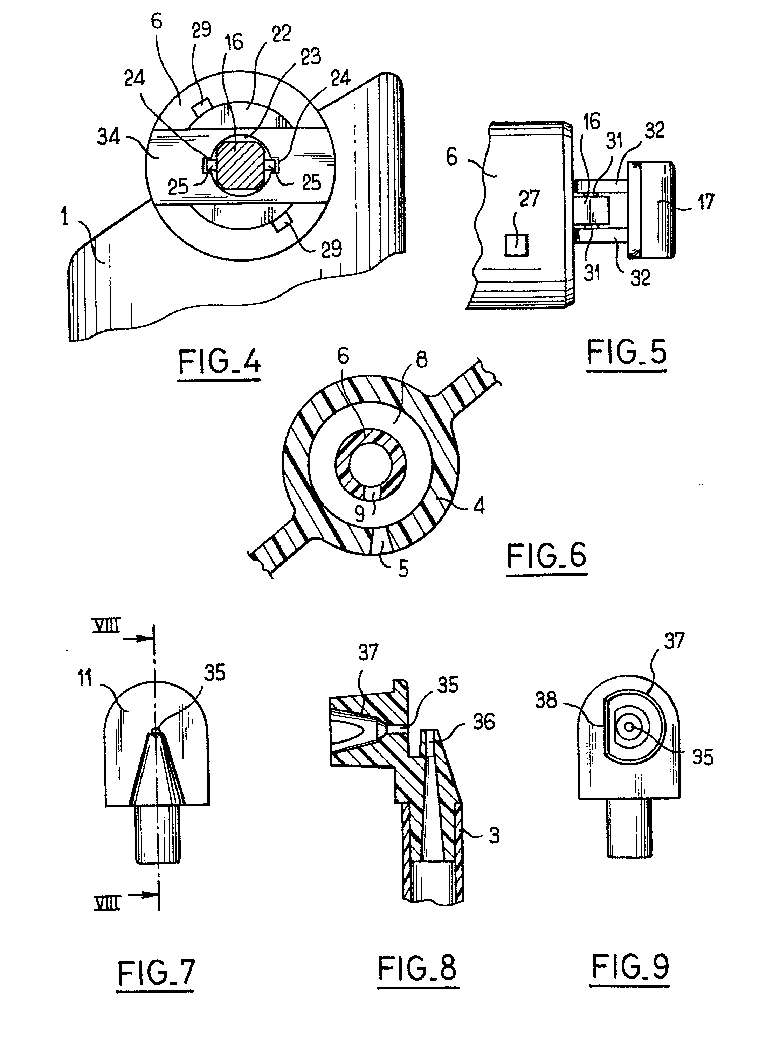

. la figure 4 est une vue en coupe selon le plan IV-IV de la figure 3;

. la figure 5 est une vue de dessus de l'extrémité de commande du distributeur;

. la figure 6 est une vue en coupe selon le plan VI-VI de la figure 3;

. la figure 7 est une vue de face de la buse de vaporisation;

. la figure 8 est une vue en coupe selon le plan VIII-VIII de la figure 7;

. la figure 9 est une vue de dos de la buse de vaporisation.

En référence aux figures 1 et 2, le vaporisateur-diffuseur à vapeur comporte d'une façon habituelle, une cheminée 1 reliée à une chaudière (non représentée) pour la formation de vapeur, généralement de la vapeur d'eau et un récipient à lotion 2 dans lequel s'étend une conduite d'aspiration 3 de la lotion.

En référence aux figures 3 à 9, le distributeur selon l'invention comporte un manchon 4 disposé à la partie supérieure de la cheminée 1 transversalement à celle-ci et comprenant un orifice d'alimentation 5 reliant l'intérieur du manchon 4 à la cheminée 1.

Un corps creux allongé 6 est disposé dans le manchon 4. Le corps creux 6 a une section circulaire avec des diamètres variés selon le point considéré et un canal central 7 de section également circulaire de diamètre variable. Au voisinage de l'orifice d'alimentation 5 de vapeur, le corps creux 6 a un diamètre extérieur légèrement inférieur au diamètre intérieur du manchon 4 et délimite ainsi une cavité annulaire 8. Le corps creux 6 comporte un orifice de liaison radial 9 entre l'intérieur du corps creux 6 et l'intérieur du manchon 4.

A l'une de ses extrémités, le corps creux 6 comporte un premier orifice de sortie 10 s'étendant transversalement et dans lequel est montée une buse de vaporisation 11. Un second orifice de sortie 12 est prévu dans le corps 6 à l'opposé du premier orifice de sortie 11 par rapport à l'orifice de liaison 9. Un clapet de séparation 13 est disposé transversalement au canal 7 entre les orifices de sortie 10 et 12 et comporte un joint d'étanchéité 14 s'appuyant sur un siège 15 formé par une partie de la paroi du canal 7. La position du clapet est commandée par un organe de commande comprenant une tige coulissante 16 solidaire du clapet 13 et reliée à une poignée de commande 17 qui sera décrite en détail plus loin.

Le corps creux 6 est monté de façon pivotante dans le manchon 4 par l'intermédiaire de joints d'étanchéité toriques 18, 19 respectivement disposés dans des gorges annulaires 20, 21 réalisées dans la paroi externe du corps creux 6 de part et d'autre de la cavité 8. Ainsi, les joints 18 et 19 réalisent non seulement un moyen de pivotement pour le corps creux 6 mais également forment une étanchéité pour la cavité 8 vis-à-vis de l'extérieur.

La position en rotation du corps creux 6 est commandée par la tige 16 reliée à la poignée 17 par l'intermédiaire de moyens de solidarisation en rotation comprenant un organe de butée 22 percé d'un canal central 23 (figure 4) comportant des rainures opposées 24 dans lesquelles s'engagent des ergots 25 portés par la tige 16. L'organe de butée 22 est disposé à l'intérieur du corps creux 6 et est rendu solidaire de celui-ci par des ergots 26 s'étendant dans des ouvertures 27 de la paroi latérale du corps creux 6. Pour faciliter l'introduction des ergots 26 dans le corps 6, les ergots 26 comprennent des rampes inclinées 28 coopérant avec des rampes complémentaires 29 réalisées à l'extrémité du corps 6.

Un ressort de rappel 30 est disposé entre la face interne de l'organe de butée 22 et le clapet 13 afin de maintenir celui-ci appliqué contre son siège 15 en position de repos.

La poignée de commande 17 est montée de façon pivotante autour d'un axe transversal à la tige de commande 16. Cet axe est réalisé par des ergots 31 disposés sur deux faces opposées de la tige 16 (figure 5) et sur lesquelles s'engagent des flasques 32 s'étendant transversalement à cet axe, ces flasques ayant un bord curviligne 33 dont la distance par rapport à l'axe de pivotement formé par les ergots 31 est variable. Le bord 33 prend appui sur une entretoise 34 formée en une seule pièce avec l'organe de butée 22 rendu solidaire du corps creux 6 comme il a été expliqué ci-dessus. L'entretoise 34 s'appuie par ses extrémités sur le pourtour du corps creux 6 et constitue un arrêt pour l'organe de butée 22 lors de son introduction dans le corps creux 6.

Dans l'exemple de réalisation représenté sur les figures, les flasques 32 ont un pourtour sensiblement rectangulaire de sorte que selon une direction sensiblement perpendiculaire au plan de la poignée 17, la distance entre les ergots 31 et le bord 33 est une distance d tandis que selon une direction sensiblement parallèle au plan de la poignée 17, la distance entre l'ergot 31 et le bord 33 est une distance D supérieure à la distance d.

La buse 11 comporte d'une façon connue en soi (figures 7 à 9) un canal d'amenée de vapeur 35 dont l'extrémité débouche au voisinage d'un canal d'aspiration de lotion 36 s'étendant sensiblement perpendiculairement au canal 35 et débouchant à son extrémité opposée au canal 35 dans le conduit 3 disposé dans le récipient de lotion 2. Afin de s'assurer que le conduit 3 plonge de façon permanente jusqu'au fond du récipient de lotion 2 il est souhaitable de s'assurer que le positionnement de la buse 11 est maintenu le plus stable possible. Pour cela, on prévoit un embout 37 comportant un méplat 38 et une forme correspondante de l'ouverture 10 du corps creux 6. Ainsi, lorsque l'embout 37 est mis en place dans l'ouverture 10, il est maintenu dans celle-ci selon une orientation prédéterminée.

Comme illustré sur la figure 3, le clapet 13 est de préférence disposé entre l'orifice de liaison 9 et le second orifice de sortie 12 et le second orifice de sortie 12 a une dimension supérieure au canal 35 de la buse 11 disposée dans le premier orifice de sortie 10.

Le fonctionnement du dispositif est le suivant: lorsque la poignée 17 est dans la position verticale représentée sur la figure 3, la distance d entre le bord 33 des flasques 32 s'appuyant sur l'organe de butée 22 et l'axe des ergots 31 est suffisamment faible pour permettre au clapet 13 de reposer sur son siège 15. La vapeur en provenance de la cheminée 1 et s'écoulant à travers les orifices 5 et 9 suit alors le trajet représenté par les flèches en trait plein et sort par le premier orifice de sortie 10. De la lotion en provenance du récipient 2 est alors aspirée par la buse de vaporisation 11. Le jet de lotion pulvérisée peut être orienté par l'utilisateur en manoeuvrant la poignée de commande 17 pour faire pivoter celle-ci autour de l'axe de la tige de commande 16.

Lorsque l'on souhaite obtenir de la vapeur seule, il suffit de faire pivoter la poignée de commande 17 autour des ergots 31 selon la flèche V de la figure 3 et la partie de bord 33 des flasques 32 qui est à une distance D des ergots 31 vient alors en contact avec l'organe de butée 22. Par ce mouvement, la tige 16 est tirée et entraîne le clapet 13 à l'encontre de l'action du ressort 30. Etant donné la différence de perte de charge importante qui existe entre le canal 35 de la buse 11 et le second orifice de sortie 12, la vapeur a naturellement tendance à s'échapper par le second orifice de sortie 12 en suivant le chemin représenté par les flèches en tiret sur la figure 3. La buse 11 n'étant pratiquement plus alimentée en vapeur, la vaporisation de lotion cesse instantanément. De la même façon que dans la position de vaporisation, l'orientation de l'orifice de sortie 12 peut être modifiée en manoeuvrant la poignée 17 pour la faire pivoter autour de l'axe de la tige 16.

On constate que le clapet 13 sert également de clapet de sécurité en cas de surpression à l'intérieur de la cheminée 1. En effet, dans ce cas le clapet 13 est repoussé à l'encontre du ressort 30 par la surpression existant dans le canal 7 et la vapeur s'échappe par le second orifice de sortie 12.

Bien entendu l'invention n'est pas limitée au mode de réalisation décrit ci-dessus et on peut y apporter des variantes d'exécution.

En particulier, bien que selon la réalisation préférée de l'invention le passage de la fonction de vaporisation à la fonction de diffusion de vapeur soit obtenu par un clapet simple associé à des dimensions différentes du premier et du second orifice de sortie, on peut prévoir un clapet double disposé de part et d'autre de l'orifice de liaison 9 et ayant des positions opposées pour fermer la liaison vers le premier orifice de sortie lors de l'ouverture de la liaison vers le second orifice de sortie et vice versa.

Bien que les moyens de solidarisation en rotation décrits comportent des ergots 25 associés à des rainures 24, on comprend que d'autres moyens peuvent être utilisés, en particulier on peut prévoir une tige 16 de section carrée et introduite dans une ouverture carrée correspondante dans l'organe de butée 22.

0172079

REVENDICATIONS

1. Distributeur pour vaporisateur-diffuseur à vapeur, caractérisé en ce qu'il comporte un manchon (4) comprenant un orifice d'alimentation de vapeur (5), un corps creux (6) disposé dans le manchon (4) et comportant un orifice de liaison (9) entre l'intérieur du corps (6) et l'intérieur du manchon (4), et au moins un premier et un second orificesde sortie (10, 12) distincts dont l'un (10) est équipé d'une buse de vaporisation (11), au moins un clapet de séparation (13) entre les orifices de sortie (10,12), et un organe de commande (16) de la position du clapet (13).

2. Distributeur conforme à la revendication 1, caractérisé en ce que le corps creux (6) est monté pivotant dans le manchon (4) et en ce que sa position est contrôlée par un organe de commande (16) commun à l'organe de commande de la position du clapet (13).

3. Distributeur conforme à la revendication 2, caractérisé en ce que l'organe de commande est une tige coulissante (16) comprenant des moyens de solidarisation (25) pour une rotation avec le corps creux (6), la tige (16) ayant une extrémité reliée au clapet (13) et l'autre extrémité portant une poignée de commande (17).

4. Distributeur conforme à la revendication 3, caractérisé en ce que la poignée de commande (17) est montée de façon pivotante autour d'un axe (31) transversal à la tige de commande (16) et comporte des flasques (32) s'étendant transversalement à cet axe, ces flasques ayant un bord curviligne (33) excentré par rapport à l'axe de pivotement (31) de la poignée de commande, ce bord (33) prenant appui sur un organe de butée (22) solidaire du corps creux (6).

5. Distributeur conforme à la revendication 3 ou à la revendication 4, caractérisé en ce que les moyens de solidarisation en rotation entre la tige (16) et le corps creux (6) comprennent des ergots (25) portés par la tige (16) s'étendant transversalement à celle-ci et coopérant avec des rainures (24) portées par l'organe de butée (22).

6. Distributeur conforme à l'une des revendications 1 à 5, caractérisé en ce que le clapet (13) est disposé entre l'orifice de liaison (9) de l'intérieur du corps creux (6) avec le manchon (4) et le second orifice de sortie (12) et en ce que le second orifice de sortie (12) a une dimension supérieure à un canal de vapeur (35) de la buse (11), la buse (11) étant portée par le premier orifice (10).

7. Distributeur conforme à la revendication 6, caractérisé en ce qu'il comporte des moyens de rappel (30) du clapet (13) vers une position de fermeture.

8. Distributeur conforme à l'une des revendication 1 à 7, caractérisé en ce que la buse de vaporisation (11) comporte un embout (37) présentant un méplat (38) et en ce que le premier orifice de sortie (10) a une forme complémentaire de cet embout (37).

0172079

FIG_1

FIG_2

FIG_3

2 / 2                    0172079

FIG_4

FIG_5

FIG_6

FIG_7          FIG_8          FIG_9

## DOCUMENTS CONSIDERES COMME PERTINENTS

| Catégorie | Citation du document avec indication. en cas de besoin. des parties pertinentes | Revendication concernee | CLASSEMENT DE LA DEMANDE (Int. Cl. 4) |
|---|---|---|---|
| A | FR-A-2 460 644 (SCHWOB) <br> * Page 2, lignes 6-11; page 5, lignes 10-18; page 7, lignes 13-19; figure 1 * <br><br> ----- | 1,3,7 | B 05 B 7/30 <br> A 61 M 11/04 |
| | | | **DOMAINES TECHNIQUES RECHERCHES (Int. Cl. 4)** <br><br> A 45 D <br> A 61 M <br> A 61 H <br> B 05 B |

Le présent rapport de recherche a été établi pour toutes les revendications

| Lieu de la recherche | Date d'achèvement de la recherche | Examinateur |
|---|---|---|
| LA HAYE | 30-10-1985 | JUGUET J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82